# FASCICULE DE BREVET EUROPEEN

(11) **EP 0 771 837 B1**
(45) Date de publication et mention de la délivrance du brevet: **19.03.2003**
(21) Numéro de dépôt: 96402292.5
(22) Date de dépôt: 29.10.1996
(51) Int. Cl.: C08J 5/18, C08K 5/00, A61F 6/04, A61B 19/04, A61L 31/00

(54) **Films d'élastomère contenant au moins une substance chimique active, leur procédé de préparation et leurs applications**
Elastomerfolien mit mindestens einem chemischen Wirkstoff, Verfahren zu ihrer Herstellung und ihre Verwendungen
Elastomeric films containing at least a chemical active substance, process for their preparation and applications thereof

(30) Priorité: 02.11.1995 FR 9512926
(43) Date de publication de la demande: 07.05.1997
(73) Titulaire: HUTCHINSON, 75008 Paris (FR)
(72) Inventeur: Hoerner, Pierre, 68180 Horbourg-Wihr (FR); Riess, Gérard, 68200 Mulhouse (FR); Busnel, René Guy, 91570 Bievres (FR); Cheymol, André, 86220 Dange Saint Romain (FR)
(74) Mandataire: Orès, Bernard

(56) Documents cités:
- EP-A- 0 306 389
- EP-A- 0 557 625
- WO-A-95/17453
- GB-A- 2 263 114

## Description

La présente invention est relative à des films d'élastomère dans lesquels sont dispersés uniformément, sous la forme d'inclusions de gel, des substances chimiques actives, telles que des anti-corrosifs, des lubrifiants ou encore des biocides à usage médical ; elle couvre également les procédés de préparation ainsi que les diverses applications de ces films.

La présente invention est également relative à des dispersions stables comportant une phase continue formée d'une solution d'élastomère dans un solvant organique et une phase dispersée comprenant au moins une substance chimique active incluse dans un gel, lesquelles dispersions sont aptes à être transformées en film d'élastomère, ainsi qu'à leur procédé de préparation.

Les différents matériaux élastomères, habituellement utilisés dans le domaine médical ou paramédical (hygiène, notamment) peuvent être modifiés, de manière à être associés à des substances chimiques ayant un effet de protection, lors de l'utilisation de ces matériaux (gants, doigtiers, préservatifs, bandes et pansements divers), comme illustré dans le Brevet européen 306 389 ou la Demande Internationale WO 95/17453, au nom de la Demanderesse, qui décrivent des films d'élastomère incorporant au moins une substance chimique active, soit sous forme d'inclusions liquides stabilisées par un copolymère séquencé ou greffé (Demande Internationale WO 95/17453), soit contenue dans des microcapsules, dont les parois se rompent sous l'action de forces de frottement ou de cisaillement (Brevet européen 306 389).

Toutefois, ces films d'élastomères présentent un certain nombre d'inconvénients :
- une dégradation dans le temps des performances des matériaux obtenus, dans le cas des microcapsules peut s'observer, d'une part en raison de réactions chimiques pouvant avoir lieu à l'intérieur de la phase dispersée, entre les substances actives et d'autre part, par diffusion de ces mêmes substances à l'extérieur de la microcapsule ;
- les microcapsules contenues dans les films d'élastomère selon le Brevet européen 306 389 peuvent présenter des difficultés à se rompre, pour libérer leurs substances actives, lors de la perforation desdits films ; et
- les films d'élastomère contenant des inclusions liquides selon la Demande Internationale WO 95/17453 mettent en oeuvre des stabilisants macromoléculaires de type copolymère greffé ou séquencé (copolymère bloc), qui sont d'un coût relativement élevé.

En conséquence, poursuivant ses recherches, la Demanderesse s'est donné pour but la mise au point d'un matériau élastomère qui répond mieux aux besoins de la pratique que l'ensemble des élastomères de l'Art antérieur, notamment en ce qui concerne la tenue au stockage et la réduction des coûts de mise en oeuvre.

En effet, la Demanderesse a maintenant trouvé qu'en incorporant des molécules chimiques actives dans un gel, la dispersion dudit gel dans un élastomère (solution et film) est stable, ne nécessite pas le recours à des agents stabilisants (agents compatibilisants) tels que les copolymères blocs ou greffés décrits dans la Demande Internationale WO 95/17453 et conduit à l'obtention d'un matériau élastomère souple.

La présente invention a pour objet un film d'élastomère contenant au moins une substance chimique active sous la forme d'une phase dispersée dans ledit élastomère, lequel film est caractérisé en ce que ladite phase dispersée est sous la forme d'un gel, réversible ou non-réversible en fonction de la température, et chargé en substance chimique active x.

Selon la Littérature (*Encyclopedia of Polymer Science and Engineering,* 2nd *édition* : Wiley Interscience; Volume 7 and Suppl. Vol), un gel peut être défini comme étant un réseau polymérique correspondant à un état de transition intermédiaire entre un liquide et un solide.

Les gels non-réversibles, en fonction de la température, sont formés par voie chimique, c'est-à-dire par réticulation covalente, alors que les gels réversibles, en fonction de la température, sont formés soit par voie physique, c'est-à-dire par interaction de type liaison hydrogène, Van der Waals ou dipôle-dipôle, soit par formation de complexe ou de domaines cristallisés.

Dans le cas des gels réversibles, une transition intervient à une température critique (Ttrans) à laquelle les interactions physiques disparaissent ; à cette transition correspond la transformation gel-liquide.

Selon la référence citée ci-dessus, la formation d'un gel est facilement détectée par changement de viscosité ou d'élasticité du système.

Conformément à l'invention, ledit gel est essentiellement constitué d'un polymère structurant c, d'un solvant dₛ et d'au moins une substance chimique active x.

Selon un mode de réalisation avantageux dudit film d'élastomère, lorsque ledit gel est réversible, il présente une température de transition gel-liquide (Ttrans), comprise entre 0°C et 120°C, de préférence comprise entre 20°C et 70°C.

Selon une disposition avantageuse de ce mode de réalisation, ledit gel réversible est essentiellement constitué d'un polymère structurant c, d'un solvant dₛ et d'au moins la substance chimique active x ; il peut contenir, en outre, un agent réticulant réversible d_{r r}.

La réticulation peut intervenir soit par interaction intermoléculaire entre les chaînes (liaisons hydrogène ou dipôle-dipôle, cristallisation partielle) du polymère structurant c, soit par addition d'un agent réticulant d_{r r} induisant la formation de liaisons physiques (complexes, liaisons hydrogène, ou force de type dipôle-dipôle) entre les chaînes de polymère structurant c.

Selon une modalité avantageuse de cette disposition, le polymère structurant c est de type hydrophile et non miscible avec la phase élastomère.

Conformément à cette modalité avantageuse de l'invention, ledit polymère structurant c est sélectionné dans le groupe constitué soit par les polymères cristallisables ou partiellement cristallisables, comme par exemple les polyéthers, de préférence parmi les polyoxyéthylènes, de masse moléculaire comprise entre 1 500 et 10⁶ Daltons et les polymères à base de polyoxyéthylène présentant, à l'état fondu, une miscibilité avec le solvant dₛ, soit par les polymères non organisés à l'état solide, comme par exemple les polymères d'origine naturelle comme la gélatine, les gommes, les pectines et les alginates, ainsi que certains dérivés cellulosiques, comme la méthylcellulose, l'hydroxyéthylcellulose, l'hydroxypropylcellulose et la carboxyméthylcellulose ; il peut en outre être éventuellement sélectionné dans le groupe constitué par les composés de synthèse comme le polyéthylèneimine, l'acide polyacrylique, les polyacrylates et polyméthacrylates tels que le poly(hydroxyéthylméthacrylate), l'alcool polyvinylique et ses dérivés, les polyvinyléthers comme le poly(vinylméthyléther).

Selon une autre modalité avantageuse de cette disposition, le solvant dₛ est sélectionné dans le groupe constitué par les polyols, de préférence parmi l'éthylène glycol, la glycérine et les polyéthylènes glycols liquides à température ambiante et de masse moléculaire comprise entre 62 (éthylène glycol) et 750 Daltons (PEG750) et l'eau, utilisés seuls ou en mélange.

Selon une autre modalité avantageuse de cette disposition, le réticulant dᵣᵣ est sélectionné dans le groupe constitué par certains dérivés du bore, comme l'acide borique ou le borax, de l'aluminium, comme l'alumine hydratée ou certains hydroxydes d'aluminium et de certains métaux bivalents comme le zinc ou le calcium ; il est utilisé comme réticulant du polymère structurant c, et agit par formation de liaisons physiques entre les chaînes du polymère structurant c.

Selon un autre mode de réalisation avantageux dudit film d'élastomère, lorsque ledit gel est de type non-réversible et ne présente par conséquent pas de température de transition Ttrans, il est essentiellement constitué d'un polymère structurant c, d'un solvant dₛ, d'un réticulant d_{r i}, (réticulant irréversible) et d'au moins une substance chimique active x.

La réticulation intervient par liaison chimique covalente, c'est dire de façon irréversible, entre les chaînes du polymère structurant c.

Selon une disposition avantageuse de ce mode de réalisation, le polymère structurant c est de type hydrophile et non miscible avec la phase élastomère.

Selon une modalité avantageuse de cette disposition, ledit polymère structurant c est sélectionné dans le groupe constitué par les polymères d'origine naturelle comme la gélatine, les gommes, les pectines et alginates, ou certains dérivés cellulosiques comme la méthylcellulose, l'hydroxyethylcellulose, l'hydroxypropylcellulose et la carboxymethylcellulose et les composés de synthèse comme le polyéthylèneimine, l'acide polyacrylique, l'alcool polyvinylique et ses dérivés, les poly(vinyléthers) comme le poly(vinylmethyléther); lesdits composés de synthèse peuvent en outre être sélectionnés parmi les polyacrylates et polyméthacrylates tels que le poly(hydroxyéthylméthacrylate).

Selon une autre disposition avantageuse de ce mode de réalisation, le composé dₛ est sélectionné dans le groupe constitué par les polyols, de préférence parmi l'éthylène glycol, la glycérine et plus généralement les polyéthylène glycols, liquides à température ambiante et de masse moléculaire comprise entre 62 (éthylène glycol) et 750 Daltons (PEG750) et l'eau, utilisés seuls ou en mélange.

Selon une autre disposition avantageuse de ce mode de réalisation, le réticulant d_{r i} est sélectionné dans le groupe constitué par les aldéhydes ou dialdéhydes à faible ou à haut poids moléculaire comme par exemple le glyoxal, mais peut être tout autre composé susceptible de réagir chimiquement avec le structurant c, comme par exemple les dérivés urée-formol ou mélamine-formol.

Selon un autre mode de réalisation avantageux dudit film d'élastomère, ladite substance chimique active x est sélectionnée parmi les composés capables de provoquer une dénaturation quasi-instantanée des protéines par simple contact, soit par réaction chimique, soit par effet physicochimique tel qu'une modification de la tension de surface. Cette famille de composés comprend entre autres les biocides.

Selon une disposition avantageuse de ce mode de réalisation, ledit biocide est un ammonium quaternaire, de préférence du diméthyldidécylammonium, des biguanides, le phtalaldéhyde, des dérivés phénoliques, le formol, des tensioactifs non-ioniques comportant au moins une séquence polyoxyéthylène, l'hexamidine, des composés iodés, par exemple le complexe iodé de la polyvinylpyrrolidone, des tensioactifs non-ioniques à activité virucide, les bichromates et hypochlorites de sodium et de potassium, utilisés seuls ou en mélange.

Selon un autre mode de réalisation avantageux dudit film d'élastomère, ledit gel comprend
∗ lorsque le gel est réticulé de manière réversible :
   - entre 25 et 98 %, de préférence entre 25 et 97 % (teneur massique) de solvant dₛ, par rapport au gel,
   - entre 1 et 74 % (teneur massique) de polymère structurant c, par rapport au gel,
   - entre 1 et 74 % (teneur massique) de ladite substance chimique active x, par rapport au gel et
   - entre 0 et 1 %, de préférence entre 0 et 0,1 % de réticulant d_{r r} (teneur massique réticulant/ensemble des constituants) ;
∗ lorsque le gel est réticulé de manière non-réversible :
   - entre 25 et 98 %, de préférence entre 25 et 97 % (teneur massique) de solvant dₛ par rapport au gel,
   - entre 1 et 74 % (teneur massique) de polymère structurant c par rapport au gel,
   - entre 1 et 74 % (teneur massique) de substance chimique active x par rapport au gel et
   - entre 0,0001 et 1 %, de préférence entre 0,0001 et 0,1 % d'agent réticulant d_{r i} (rapport massique réticulant / ensemble des constituants).

L'élastomère peut être sélectionné, et ce, de manière non limitative, parmi le polybutadiène, le polyisoprène, les copolymères SBR *(Styrène Butadiene Rubber),* NBR *(Nitrile Butadiene Rubber),* SBS (*Styrene Butadiene Styrène),* SIS (*Styrene Isoprene Styrène)* ou SEBS *(Styrène Ethylene Butylene Styrene*).

Selon un autre mode de réalisation avantageux dudit film d'élastomère, les proportions massiques de l'élastomère et du gel sont les suivantes :
- élastomère : entre 20 et 95 % (teneur massique), exprimée par rapport au film final, et
- gel : entre 5 et 80 % (teneur massique), exprimée par rapport au film final.

La présente invention a également pour objet un procédé de préparation dudit film d'élastomère, caractérisé en ce qu'il comprend :
**1) la préparation d'une dispersion d'un gel qui comprend :**
   - la préparation d'une phase A par dissolution de l'élastomère dans un solvant organique a ;
   - la préparation d'une phase B (gel) et la dispersion de la phase B dans la phase A, selon l'une des modalités suivantes :
      ∗ lorsque le gel est réticulé de manière réversible, la phase B peut se préparer selon deux façons différentes, selon l'utilisation ou non de réticulant d_{r r} :
         (i) dans le cas où le gel se forme spontanément par liaisons de type physique, la phase B se prépare par mélange d'au moins une substance chimique active x dans un solvant dₛ, suivi du mélange de la solution obtenue avec un polymère c, à une température supérieure à la température de transition Ttrans, correspondant à la transition gel-liquide, laquelle température Ttrans est comprise entre 0°C et 120°C, de préférence entre 20°C et 120°C, suivi de la dispersion de la phase B dans la phase A, à une température supérieure à Ttrans ; la taille des inclusions de gel peut s'ajuster en fonction de la vitesse d'agitation et de la vitesse de refroidissement de la dispersion ;
         (ii) dans le cas où l'addition d'un réticulant d_{r r} est nécessaire pour la formation du gel réversible, la phase B se prépare par mélange d'au moins une substance chimique active x dans un solvant dₛ, suivi du mélange de la solution obtenue avec un polymère structurant c. Cette phase B est dispersée dans la phase A avant ajout du réticulant d_{r r}.
      ∗ lorsque le gel est réticulé de manière non-réversible, la phase B s'obtient par mélange d'au moins une substance chimique active x dans un solvant dₛ, suivi du mélange de la solution obtenue avec un polymère structurant c, puis cette phase B est dispersée dans la phase A avant ajout de l'agent réticulant d_{r i} et
2) **l'évaporation du solvant organique a**, pour l'obtention d'un film d'élastomère renfermant sous forme de dispersion stable, des inclusions de gel.

Dans le cas d'un gel réticulé de manière réversible, l'évaporation du solvant s'effectue à une température inférieure à Ttrans, et le film d'élastomère renferme des inclusions de gel comprenant le polymère c, le solvant dₛ, éventuellement le réticulant d_{r r}, et est chargé en substance chimique active x.

Dans le cas d'un gel réticulé de manière non-réversible, l'évaporation du solvant s'effectue à une température, choisie en fonction du temps d'évaporation désiré, et comprise entre 0 et 120°C et le film d'élastomère renferme des inclusions de gel comprenant le polymère c, le solvant dₛ, le réticulant d_{r i}, et est chargé en substance chimique active x.

Selon un mode de mise en oeuvre avantageux dudit procédé, le solvant apolaire ou peu polaire a est sélectionné parmi les hydrocarbures aromatiques, aliphatiques et alicycliques, par exemple des hydrocarbures paraffiniques, le cyclohexane, le benzène, le toluène, le xylène, la tétraline, la décaline, des coupes pétrolières (composées majoritairement d'hydrocarbures aliphatiques cycliques et caractérisées par leur température d'ébullition), ou un mélange de ceux-ci.

Conformément au procédé selon l'invention: le polymère structurant c, le solvant dₛ, les agents réticulants d_{r r} ou d_{r i} et la substance chimique active x sont tels que définis ci-dessus.

La présente invention a également pour objet une dispersion, apte à être utilisée pour la préparation d'un film d'élastomère conforme à l'invention, caractérisée en ce qu'elle comprend :
- une phase A contenant un élastomère dissous dans un solvant organique a (phase hydrophobe ou apolaire), tel que défini ci-dessus, dans laquelle est dispersée, sous forme d'un gel réversible ou non-réversible, une phase B comprenant une substance chimique active x incluse dans un polymère structurant c, un solvant dₛ, et éventuellement un agent réticulant d_{r r} ou d_{r i}, tels que définis ci-dessus.

De telles dispersions présentent une stabilité à la sédimentation d'au moins 12 heures à température ambiante alors que la stabilité des films au stockage (c'est à dire conservation de l'efficacité virucide et de la structure) est largement supérieure à une année.

La présente invention a également pour objet un procédé de préparation desdites dispersions stables, caractérisé en ce qu'il comprend :
- la préparation d'une phase A, par dissolution de l'élastomère dans un solvant organique a ;
- la préparation d'une phase B (gel), qui peut se réaliser selon deux modalités différents, selon le type de gel :
   ∗ dans le cas où le gel est réticulé de manière réversible, c'est à dire présente une température de transition Ttrans gel-liquide, la phase B s'obtient :
      - si le gel se forme spontanément par liaisons de type physique, la phase B se prépare par mélange d'au moins une substance chimique active x dans un solvant dₛ, suivi du mélange de la solution obtenue avec un polymère structurant c, à une température supérieure à la température de transition Ttrans, correspondant à la transition gel-liquide, laquelle température Ttrans est comprise entre 20°C et 120°C. Cette phase B est ensuite dispersée dans la phase A à une température supérieure à Ttrans. La taille des inclusions de gel est contrôlée par la vitesse d'agitation et par la vitesse de refroidissement ;
      - dans le cas où l'addition d'un agent réticulant d_{r r} est nécessaire pour la formation du gel réversible, la phase B se prépare par mélange d'au moins une substance chimique active x dans un solvant dₛ, suivi du mélange de la solution obtenue avec un polymère structurant c. Cette phase B est dispersée dans la phase A avant ajout de l'agent réticulant d_{r r}.
   ∗ dans le cas où le gel est réticulé de manière non-réversible, la phase B s'obtient par mélange d'au moins une substance chimique active x dans un solvant dₛ, suivi du mélange de la solution obtenue avec un polymère structurant c ; cette phase B est dispersée dans la phase A, avant ajout de l'agent réticulant d_{r i}.

La présente invention a également pour objet les différentes applications du film d'élastomère selon l'invention en tant que revêtement de supports notamment en élastomère ou en plastique ou de surmoulage d'un joint frottant.

Outre les dispositions qui précèdent, l'invention comprend encore d'autres dispositions, qui ressortiront de la description qui va suivre, qui se réfère à des exemples de mise en oeuvre du procédé objet de la présente invention, ainsi qu'aux dessins annexés, dans lesquels :
- la figure 1 illustre la structure de la dispersion de gel en milieu solvant ;
- la figure 2 illustre la structure de la dispersion du gel dans le film sec ;
- les figures 3 et 4 illustrent les combinaisons du mélange polymère c, substance active x et solvant dₛ et les délimitations du domaine d'existence du gel (par exemple POE 5000, Bardac® et PEG400 (figure 3); gélatine/eau, Bardac® et PEG400 (figure 4)) conduisant à un gel à température ambiante (diagramme ternaire) ;

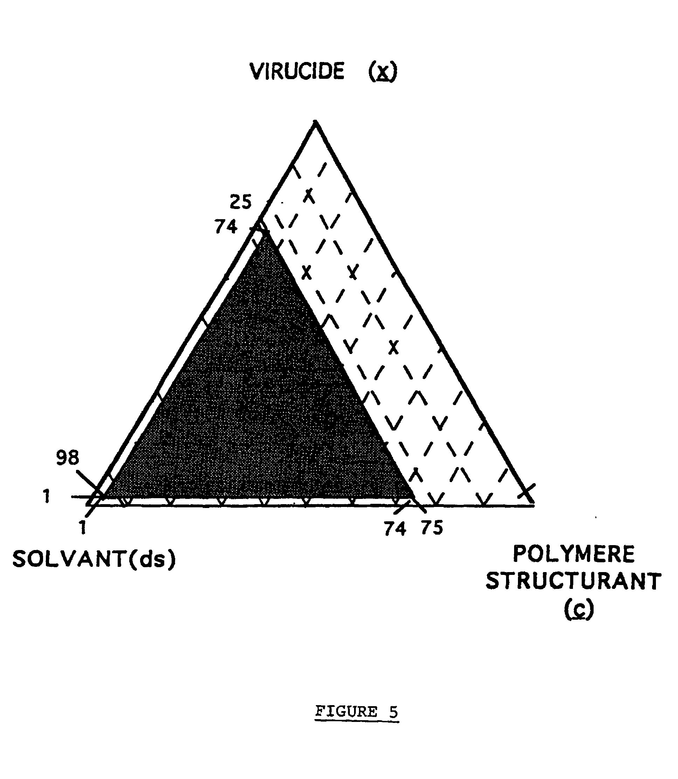

- la figure 5 illustre la délimitation du domaine d'existence d'un gel comportant un polymère structurant c, une substance chimique active x et un solvant dₛ. Cette figure représente le cas d'un gel réversible sans agent réticulant d_{r r} ; dans cette figure, le domaine grisé correspond au domaine de composition revendiqué.

Il doit être bien entendu, toutefois, que ces exemples sont donnés uniquement à titre d'illustration de l'objet de l'invention, dont ils ne constituent en aucune manière une limitation.

### EXEMPLE 1 : Film d'élastomère selon l'invention à base de polyisoprène contenant des inclusions d'un gel réversible comprenant en tant que polymère structurant c, du POE 5000 et ne comportant pas d'agent réticulant d_{r r}.

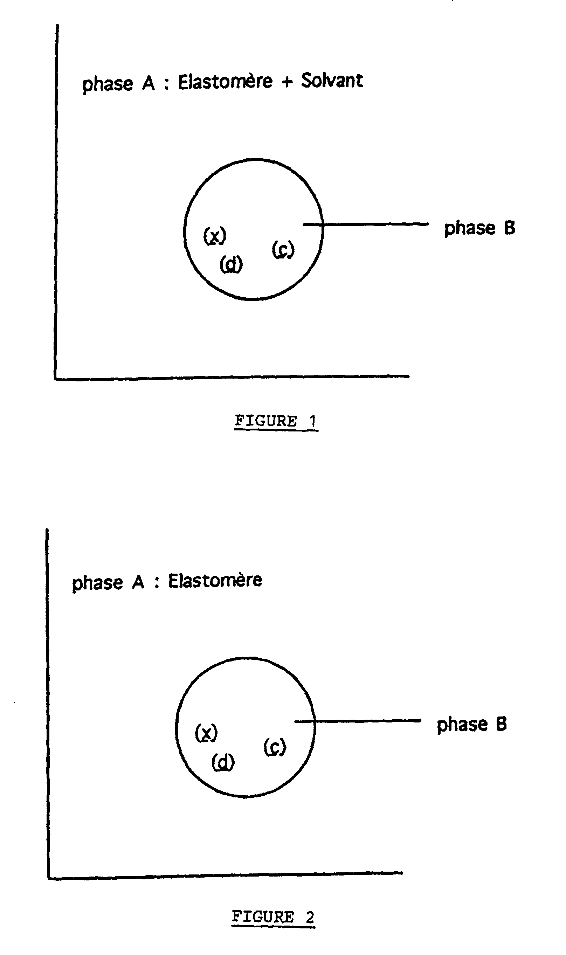

i) Préparation d'une dispersion stable de gel, conforme à l'invention.
   - Préparation de la phase continue (phase A)
      On dissout, sous agitation, du polyisoprène (PI) de synthèse, de masse moléculaire comprise entre 1 000 000 et 2 000 000 Daltons dans du cyclohexane, de manière à obtenir une solution à 7 % en masse en PI.
   - Préparation de la phase dispersée (phase B) :
      On dissout, sous agitation, du Bardac® (chlorure de diméthyldidécylammonium) dans du polyéthylène glycol de masse moléculaire 400 Daltons (PEG 400), de manière à obtenir une solution à 10% en Bardac®.
      Cette solution est portée à 60°C, et on ajoute sous agitation du polyoxyéthylène de masse moléculaire 5000 Daltons (POE 5000), jusqu'à obtention d'une solution limpide contenant 10 % en poids en POE 5000.
      La température du milieu (60°C) correspond à une température supérieure à la température de transition gel-liquide (Ttrans), qui est déterminée par la variation brusque de la viscosité du mélange à cette température et est, dans ce cas 45-50°C.
   - Préparation de la dispersion de gel :
      A la phase A, c'est à dire à la solution de PI dans le cyclohexane, on ajoute à 60°C sous agitation, 5,52 % de phase B (constituée d'un mélange de POE 5000, PEG400 et de Bardac®) en masse par rapport à la phase A.
      A température ambiante (20°C), on obtient ainsi une dispersion stable au moins 12 heures d'un gel virucide, dont la taille des particules est en moyenne de 3 à 5 µm.
      La taille des inclusions est ajustable par contrôle de la vitesse d'agitation et de la vitesse de refroidissement après dispersion.
      La structure de la dispersion de gel est illustrée à la figure 1.
ii) Préparation d'un film d'élastomère
   On évapore le solvant organique de la dispersion précédemment réalisée à 20°C, sous pression atmosphérique.
   On obtient un film élastomère renfermant des inclusions de gel dont la taille est en moyenne de 3 à 5 µm.
   Les inclusions sont dispersées de manière uniforme dans l'élastomère et se trouvent sous forme de gel, à température ambiante.
   Elles représentent environ 40 % en masse du matériau et leur composition est celle de la phase B, définie ci-dessus, à savoir : 10 % de POE 5000, 9 % de Bardac® et 81 % de PEG400.
   La structure de la dispersion du gel dans le film est illustrée à la figure 2.

### EXEMPLE 2 : Film d'élastomère selon l'invention, à base de SEBS, contenant des inclusions d'un gel réversible comprenant en tant que polymère structurant c, du POE 5000 et ne comportant pas d'agent réticulant d_{r r}

On opère comme à l'exemple 1, en remplaçant le polyisoprène par du SEBS (*Styrene Ethylene Butylène Styrene*) de synthèse (de masse moléculaire totale de 53 000 Daltons et dont la teneur pondérale en PS est de 29%).

Le SEBS est dissous sous agitation et à 30°C dans un solvant à prédominance naphténique, de manière à obtenir une solution à 15 % en masse en SEBS.

La préparation de la phase B (gel virucide) et sa dispersion dans la phase A définie ci dessus, sont réalisées selon la méthode décrite dans l'exemple 1.

### EXEMPLE 3 : Film d'élastomère selon l'invention, à base de SEBS, contenant des inclusions d'un gel réversible comprenant en tant que polymère structurant c, du POE 5000 et ne comportant pas d'agent réticulant d_{r r}

On opère comme à l'exemple 2, en faisant cependant varier les proportions du mélange constitutif du gel (POE 5000, PEG400 et Bardac®).

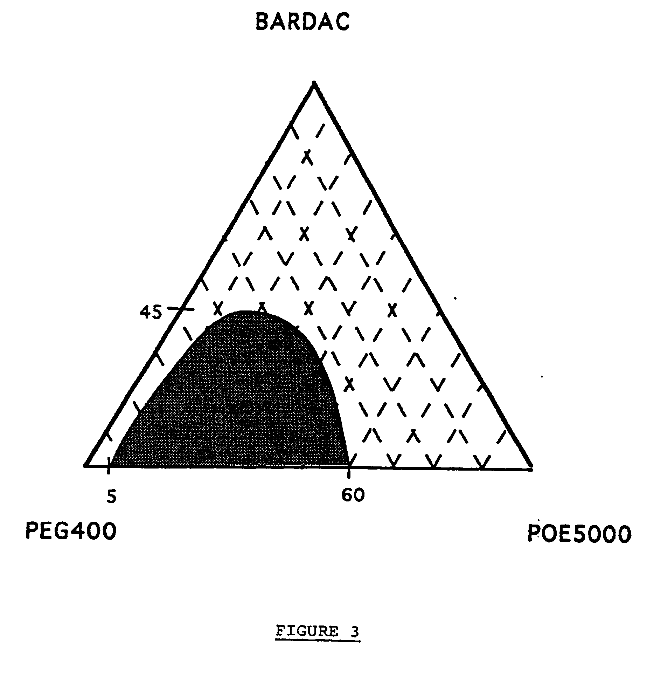

Les combinaisons du mélange conduisant à un gel à température ambiante sont délimitées par le diagramme de phase illustré à la figure 3, dans lequel le domaine grisé correspond au domaine d'existence du gel à 20°C.

La teneur en PEG400 est diminuée de 81 % à 63,6 % puis à 45,5 % par rapport à la phase B, alors que la teneur en POE 5000 est augmentée respectivement à 27,4 % puis à 45,5 %, la teneur massique en Bardac® étant maintenue constante à 9 % (également exprimée en teneur massique par rapport à la phase B).

Lesdites combinaisons conduisent à un gel, qui peut être dispersé selon les conditions de l'exemple 1.

Par ailleurs, le Bardac® peut être utilisé seul ou en mélange avec d'autres composés ayant une activité virucide, par exemple le digluconate de chlorhexidine.

En remplaçant, dans la composition du gel ci-dessus, le Bardac® par l'un des mélanges suivants :
. 90 % de Bardac® 2270E à 70 % de principe actif
   + 10 % de digluconate de chlorhexidine
   ou
. 80 % de Bardac® 2270E à 70 % de principe actif
   + 20 % de digluconate de chlorhexidine
   ou
. 75 % de Bardac® 2270E à 70 % de principe actif
   + 25 % de digluconate de chlorhexidine,
   on obtient un gel à température ambiante, conduisant, selon les conditions de l'exemple 1, à une dispersion stable.

### EXEMPLE 4 : Film d'élastomère selon l'invention, à base de SEBS, contenant des inclusions d'un gel réversible comprenant en tant que polymère structurant c, du POE m, m étant compris entre 2 000 et 100 000 et ne comportant pas d'agent réticulant d_{r r}

On opère comme à l'exemple 2, en remplaçant le POE 5000 par un autre POE, de masse moléculaire différente, soit POE m, m représentant la masse moléculaire totale et étant comprise entre 2 000 et 100 000.

Dans ces conditions, on obtient une dispersion stable de taille comprise entre 1 et 25 µm ; le film obtenu après évaporation du solvant renferme une dispersion homogène d'inclusions de gel, de taille comprise entre 1 et 25 µm.

### EXEMPLE 5 : Film d'élastomère selon l'invention à base de polyisoprène ou de SEBS, contenant des inclusions d'un gel réversible comprenant en tant que polymère structurant c, de la gélatine et ne comportant pas d'agent réticulant d_{r r}.

On opère comme à l'exemple 1 ou à l'exemple 2, en remplaçant le POE 5000 par de la gélatine, issue par traitement acide du collagène et d'indice de Bloom de 175.
- Préparation de la phase dispersée (phase B) :
   On dissout, sous agitation à 60°C de la gélatine dans un mélange de Bardac® et d'éthylène glycol comprenant 25 % en masse de Bardac®.
   La solution limpide formée contient 14,3 % en masse de gélatine.
   La température du milieu (60°C) correspond à une température supérieure à la température de transition gel-liquide, qui est déterminée par variation brusque de la viscosité du mélange à cette température et est, dans le cas présent de 30-35°C.
- Préparation de la dispersion de gel
   De façon analogue à celle de l'exemple 1, on ajoute sous agitation et à 60°C, 5,52 % en masse de phase B par rapport à la phase A.

A température ambiante, on obtient une dispersion stable au moins 12 heures de gel virucide, dont la taille des particules est en moyenne de 1 à 10 µm.

Le film d'élastomère, obtenu par évaporation du solvant organique à 20°C sous pression atmosphérique renferme des inclusions de gel dont la taille est en moyenne de 1 à 10 µm. Leur composition est celle de la phase B, à savoir : 14,3 % de gélatine, 64,3 % d'éthylène glycol, 21,4 % de Bardac®.

### EXEMPLE 6 : Film d'élastomère selon l'invention à base de polyisoprène ou de SEBS, contenant des inclusions d'un gel réversible comprenant en tant que polymère structurant c, de la gélatine et ne comportant pas d'agent réticulant d_{r r}.

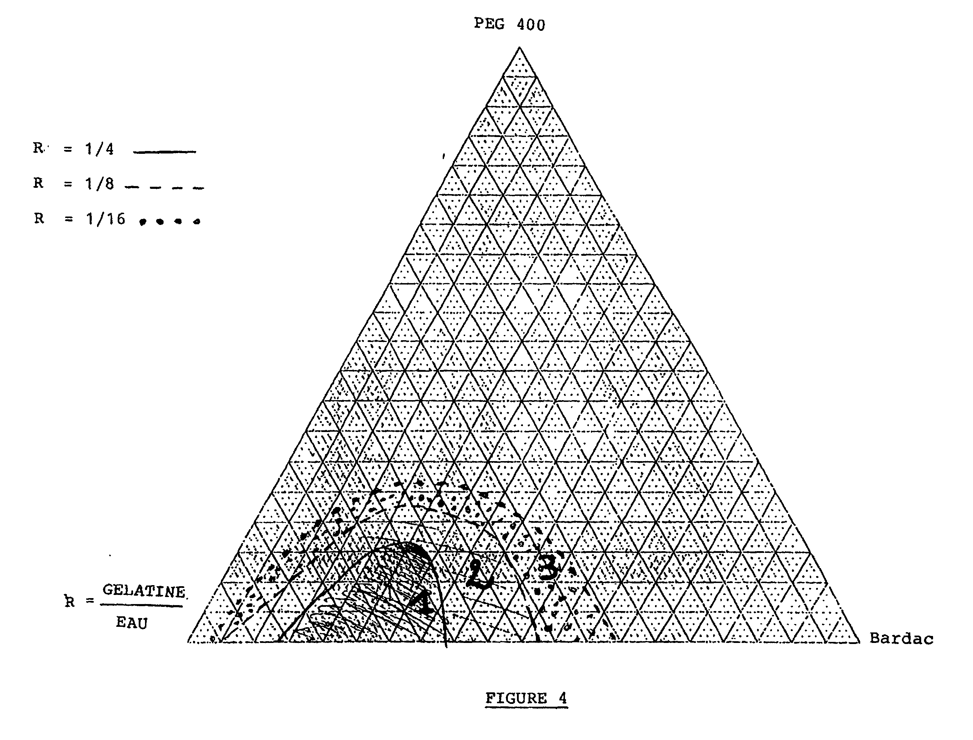

On opère comme à l'exemple 5, en remplaçant le Bardac® par un complexe iodé de polyvinylpyrrolidone (commercialisé par la Société Aldrich, sous la référence 86,056-5).
- Préparation de la phase dispersée (phase B) :
   On dissout, sous agitation à 60°C de la gélatine dans un mélange contenant du complexe iodé de polyvinylpyrrolidone, de l'eau et du polyéthylène glycol 400 dans les proportions massiques suivantes : 14,5 % de gélatine, 72,7 % d'eau, 5,5 % de complexe iodé et 7,3 % de polyéthylène glycol 400.
   La solution limpide rouge sang formée contient 14,5 % en masse de gélatine.
   La température du milieu (60°C) correspond à une température supérieure à la température de transition gel-liquide, qui est déterminée par variation brusque de la viscosité du mélange à cette température et est, dans le cas présent de 30-35°C.
   Les combinaisons du mélange conduisant à un gel à température ambiante sont délimitées par le diagramme de phase illustré à la figure 4, dans lequel les domaines grisés 1, 2 et 3 correspondent aux domaines de composition revendiqués : le domaine 1, délimité par un trait continu, correspond à un rapport R massique gélatine/eau de 1/4, le domaine 2, délimité par un trait discontinu, correspond à un rapport R massique gélatine/eau de 1/8 et le domaine 3, délimité par des points, correspond à un rapport R massique gélatine/eau de 1/16.
   La préparation de la dispersion de gel se fait de façon analogue à celle de l'exemple 5, c'est-à-dire que l'on disperse 5,52% en masse de phase B ainsi constituée par rapport à la phase A ; la dispersion ainsi réalisée est stable 12 heures et le film renferme des inclusions de taille comprise entre 1 et 25 µm.

### EXEMPLE 7 :

On opère comme aux exemples 5 et 6, en utilisant également les mêmes composants. On fait cependant varier la proportion de phase B de 10 % à 80 %, par rapport au film final. Dans ces conditions, on obtient une dispersion stable dont la taille des particules de la phase dispersée varie entre 0,1 et 25 µm.

### EXEMPLE 8 : Film d'élastomère selon l'invention à base de polyisoprène ou de SEBS, contenant des inclusions d'un gel réversible comprenant en tant que polymère structurant c, de l'alcool polyvinylique et comportant un agent réticulant d_{r r} : borax.

Dans cet exemple, le polymère structurant c est de l'alcool polyvinylique de type Mowiol® 28-99, caractérisé par une masse moléculaire de 88 000 Daltons et un pourcentage d'hydrolyse de 99 %. Sa viscosité en solution à 4 % dans l'eau est de 28 mPa.s.

La préparation de la phase A est analogue à celle de l'exemple 1 ou de l'exemple 2.
- Préparation de la phase dispersée (phase B) :
   On dissout sous agitation et à 80°C du Mowiol® dans de l'eau (solvant dₛ), pour obtenir une solution à 2,5 % de Mowiol®.
   A cette solution, est ajouté, sous agitation, du Triton X100® (substance chimique active x), jusqu'à l'obtention d'une solution limpide contenant 10 % de Triton® (exprimé par rapport à la solution de Mowiol®).
- Préparation de la dispersion de gel :
   De façon analogue à l'exemple 1 ou l'exemple 2, on ajoute sous agitation et à 30°C, 5,52% en masse de phase B par rapport à la phase A. Enfin, le gel est réalisé par ajout de borax, en teneur massique de 5% (exprimé par rapport à la solution de Mowiol®).
   A température ambiante, on obtient une dispersion de gel virucide stable au moins 12 heures, et dont la taille des particules est en moyenne de 1 à 5 µm.
   Le film d'élastomère, obtenu par évaporation du solvant organique à 20°C sous pression atmosphérique renferme des inclusions de gel dont la taille est en moyenne de 1 à 5 µm.

### EXEMPLE 9 : Film d'élastomère selon l'invention à base de polyisoprène ou de SEBS, contenant des inclusions d'un gel non-réversible comprenant en tant que polymère structurant c, de l'alcool polyvinylique et comportant un agent réticulant d_{r i} : glutaraldéhyde.

On opère comme à l'exemple 8, en utilisant également les mêmes composants dans les mêmes proportions.

On fait cependant varier le type d'agent réticulant, en remplaçant le borax par du glutaraldéhyde (agent réticulant irréversible).
- Préparation de la phase dispersée (phase B) :
   On dissout sous agitation et à 80°C du Mowiol® dans de l'eau, pour obtenir une solution à 2,5 % de Mowiol®.
   A cette solution est ajouté, sous agitation, du Triton X100® jusqu'à l'obtention d'une solution limpide contenant 10 % de Triton® (exprimé par rapport à la solution de Mowiol®).
- Préparation de la dispersion de gel :
   De façon analogue à celle de l'exemple 1, on ajoute sous agitation et à 50°C, 5,52 % en masse de phase B par rapport à la phase A.
   Le gel est ensuite réalisé par réticulation de l'alcool polyvinylique en milieu acide, par ajout, sous agitation, de 0,475 % (teneur massique exprimée par rapport à la solution de Mowiol®) d'une solution à 0,005% de glutaraldéhyde (en masse) dans un mélange eau : acide chlorhydrique comprenant 9 parties d'eau pour une partie d'acide molaire.
   A température ambiante, on obtient une dispersion stable au moins 12 heures de gel virucide, dont la taille des particules est en moyenne de 1 à 5 µm.
   Le film d'élastomère, obtenu par évaporation du solvant organique à 20°C sous pression atmosphérique, renferme des inclusions de gel dont la taille est en moyenne de 1 à 5 µm.

### EXEMPLE 10 : Préparation, de gants, doigtiers ou préservatifs revêtus d'un film d'élastomère conforme à l'invention.

En tant que revêtement de matériau élastomère, le film d'élastomère conforme à l'invention est rapporté sur une couche de matériau élastomère préalablement façonnée à la forme voulue par les techniques usuelles de fabrication, comme suit:

On dépose, par exemple, sur une forme en céramique, en verre ou en matériau analogue définissant le gant, le doigtier ou le préservatif à fabriquer, au moins la couche d'élastomère selon l'invention. on procède notamment comme décrit dans le Brevet européen 0 306 389.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

1. Film d'élastomère contenant au moins une substance chimique active sous la forme d'une phase dispersée dans ledit élastomère, lequel film est **caractérisé en ce que** ladite phase dispersée est sous la forme d'un gel, réversible ou non-réversible en fonction de la température, et chargé en substance chimique active x.

2. Film d'élastomère selon la revendication 1, **caractérisé en ce que** lorsque ledit gel est réversible en fonction de la température, il présente une température de transition gel-liquide (Ttrans), comprise entre 0°C et 120°C, de préférence comprise entre 20°C et 70°C.

3. Film d'élastomère selon la revendication 1 ou la revendication 2, **caractérisé en ce que** ledit gel réversible est essentiellement constitué d'un polymère structurant c, d'un solvant dₛ et de la substance chimique active x.

4. Film d'élastomère selon la revendication 3, **caractérisé en ce qu'**il contient, en outre, un agent réticulant réversible d_{r r}.

5. Film d'élastomère selon la revendication 3 ou la revendication 4, **caractérisé en ce que** le gel comprend :
- entre 25 et 97 % (teneur massique) de solvant dₛ, par rapport au gel,
- entre 1 et 74 % (teneur massique) de polymère structurant c, par rapport au gel,
- entre 1 et 74 % (teneur massique) de ladite substance chimique active x, par rapport au gel et
- entre 0 et 1 % de réticulant d_{r r} (teneur massique réticulant/ensemble des constituants).

6. Film d'élastomère selon l'une quelconque des revendications 1 à 5, **caractérisé en ce que** le polymère structurant c est de type hydrophile et non miscible avec la phase élastomère.

7. Film d'élastomère selon l'une quelconque des revendications 1 à 6, **caractérisé en ce que** ledit polymère structurant c est sélectionné dans le groupe constitué soit par les polymères cristallisables ou partiellement cristallisables, comme par exemple les polyéthers, de préférence parmi les polyoxyéthylènes, de masse moléculaire comprise entre 1 500 et 10⁶ Daltons et les polymères à base de polyoxyéthylène présentant, à l'état fondu, une miscibilité avec le solvant dₛ, soit par les polymères non organisés à l'état solide, comme par exemple les polymères d'origine naturelle comme la gélatine, les gommes, les pectines et les alginates, ainsi que certains dérivés cellulosiques, comme la méthylcellulose, l'hydroxyéthylcellulose, l'hydroxypropylcellulose et la carboxyméthylcellulose et les composés de synthèse comme le polyéthylèneimine, l'acide polyacrylique, les polyacrylates et polyméthacrylates tels que le poly(hydroxyéthylméthacrylate), l'alcool polyvinylique et ses dérivés, les polyvinyléthers comme le poly(vinylméthyléther).

8. Film d'élastomère selon l'une quelconque des revendications 1 à 7, **caractérisé en ce que** le solvant dₛ est sélectionné dans le groupe constitué par les polyols, de préférence parmi l'éthylène glycol, la glycérine et les polyéthylènes glycols liquides à température ambiante et de masse moléculaire comprise entre 62 (éthylène glycol) et 750 Daltons (PEG750) et l'eau, utilisés seuls ou en mélange.

9. Film d'élastomère selon l'une quelconque des revendications 1 à 8, **caractérisé en ce que** le réticulant d_{r r} est sélectionné dans le groupe constitué par certains dérivés du bore, comme l'acide borique ou le borax, de l'aluminium, comme l'alumine hydratée ou certains hydroxydes d'aluminium et de certains métaux bivalents comme le zinc ou le calcium.

10. Film d'élastomère selon la revendication 1, **caractérisé en ce que** lorsque ledit gel est de type non-réversible, il est essentiellement constitué d'un polymère structurant c, d'un solvant dₛ, d'un réticulant d_{r i}, et d'au moins une substance chimique active x.

11. Film d'élastomère selon la revendication 10, **caractérisé en ce que** le gel comprend :
- entre 25 et 97 % (teneur massique) de solvant dₛ par rapport au gel,
- entre 1 et 74 % (teneur massique) de polymère structurant c par rapport au gel,
- entre 1 et 74 % (teneur massique) de substance chimique active x par rapport au gel et
- entre 0,0001 et 1 % d'agent réticulant d_{r i} (rapport massique réticulant / ensemble des constituants).

12. Film d'élastomère selon la revendication 10, **caractérisé en ce que** le polymère structurant c est de type hydrophile et non miscible avec la phase élastomère.

13. Film d'élastomère selon l'une quelconque des revendications 10 à 12, **caractérisé en ce que** ledit polymère structurant c est sélectionné dans le groupe constitué par les polymères d'origine naturelle comme la gélatine, les gommes, les pectines et alginates, ou certains dérivés cellulosiques comme la méthylcellulose, l'hydroxyethylcellulose, l'hydroxypropylcellulose et la carboxymethylcellulose et par les composés de synthèse comme le polyéthylèneimine, l'acide polyacrylique, les polyacrylates et polyméthacrylates tels que le poly(hydroxyéthylméthacrylate), l'alcool polyvinylique et ses dérivés, les poly(vinyléthers) comme le poly(vinylmethyléther).

14. Film d'élastomère selon l'une quelconque des revendications 10 à 13, **caractérisé en ce que** le composé dₛ est sélectionné dans le groupe constitué par les polyols, de préférence parmi l'éthylène glycol, la glycérine et plus généralement les polyéthylènes glycols, liquides à température ambiante et de masse moléculaire comprise entre 62 (éthylène glycol) et 750 Daltons (PEG750) et l'eau, utilisés seuls ou en mélange.

15. Film d'élastomère selon l'une quelconque des revendications 10 à 14, **caractérisé en ce que** le réticulant, dᵣᵢ est sélectionné dans le groupe constitué par les aldéhydes ou dialdéhydes à faible ou à haut poids moléculaire, les dérivés urée-formol et mélamine-formol.

16. Film d'élastomère selon l'une quelconque des revendications 1 à 15, **caractérisé en ce que** ladite substance chimique active x est sélectionnée parmi les composés capables de provoquer une dénaturation quasi-instantanée des protéines par simple contact, soit par réaction chimique, soit par effet physicochimique, tel qu'une modification de la tension de surface.

17. Film d'élastomère selon la revendication 16, **caractérisé en ce que** ladite substance chimique active x est sélectionnée parmi les biocides.

18. Film d'élastomère selon la revendication 16 ou la revendication 17, **caractérisé en ce que** ledit biocide est un ammonium quaternaire, de préférence du diméthyldidécylammonium, des biguanides, le phtalaldéhyde, des dérivés phénoliques, le formol, des tensio-actifs non-ioniques comportant au moins une séquence polyoxyéthylène, l'hexamidine, des composés iodés, des tensio-actifs non-ioniques à activité virucide, les bichromates et hypochlorites de sodium et de potassium, utilisés seuls ou en mélange.

19. Film d'élastomère selon l'une quelconque des revendications 1 à 18, **caractérisé en ce que** l'élastomère est sélectionné parmi le polybutadiène, le polyisoprène, les copolymères SBR *(Styrène Butadiene Rubber),* NBR (*Nitrile Butadiene Rubber*), SBS *(Styrène Butadiene Styrene*), SIS *(Styrène Isoprene Styrène)* ou SEBS (*Styrène Ethylene Butylène Styrene*).

20. Film d'élastomère selon l'une quelconque des revendications 1 à 19, **caractérisé en ce que** les proportions massiques de l'élastomère et du gel sont les suivantes :
- élastomère : entre 20 et 95 % (teneur massique), exprimée par rapport au film final, et
- gel : entre 5 et 80 % (teneur massique), exprimée par rapport au film final.

21. Procédé de préparation du film d'élastomère selon l'une quelconque des revendications 1 à 20, **caractérisé en ce qu'**il comprend :
1) **la préparation d'une dispersion d'un gel qui comprend :**
- la préparation d'une phase A par dissolution de l'élastomère dans un solvant organique a ;
- la préparation d'une phase B (gel) et la dispersion de la phase B dans la phase A, selon l'une des modalités suivantes
∗ lorsque le gel est réticulé de manière réversible, la phase B peut se préparer selon deux façons différentes, selon l'utilisation ou non de réticulant d_{r r} :
(i) dans le cas où le gel se forme spontanément par liaisons de type physique, la phase B se prépare par mélange d'au moins une substance chimique active x dans un solvant dₛ, suivi du mélange de la solution obtenue avec un polymère c, à une température supérieure à la température de transition Ttrans, correspondant à la transition gel-liquide, laquelle température Ttrans est comprise entre 20°C et 120°C, suivi de la dispersion de la phase B dans la phase A, à une température supérieure à Ttrans ;
(ii) dans le cas où l'addition d'un réticulant d_{r r} est nécessaire pour la formation du gel réversible, la phase B se prépare par mélange d'au moins une substance chimique active x dans un solvant dₛ, suivi du mélange de la solution obtenue avec un polymère structurant c, puis de la dispersion de cette phase B dans la phase A avant ajout du réticulant d_{r r} ;
∗ lorsque le gel est réticulé de manière non-réversible, la phase B s'obtient par mélange d'au moins une substance chimique active x dans un solvant dₛ, suivi du mélange de la solution obtenue avec un polymère structurant c, puis cette phase B est dispersée dans la phase A avant ajout de l'agent réticulant d_{r i} et
2) **l'évaporation du solvant organique a,** pour l'obtention d'un film d'élastomère renfermant sous forme de dispersion stable, des inclusions de gel.

22. Procédé de préparation selon la revendication 21, **caractérisé en ce que** dans le cas d'un gel réticulé de manière réversible, l'évaporation du solvant s'effectue à une température inférieure à Ttrans.

23. Procédé de préparation selon la revendication 21, **caractérisé en ce que** dans le cas d'un gel réticulé de manière non-réversible, l'évaporation du solvant s'effectue à une température choisie en fonction du temps d'évaporation désiré et comprise entre 0 et 120°C.

24. Procédé selon l'une quelconque des revendications 21 à 23, **caractérisé en ce que** le solvant apolaire ou peu polaire a est sélectionné parmi les hydrocarbures aromatiques, aliphatiques et alicycliques, par exemple des hydrocarbures paraffiniques, le cyclohexane, le benzène, le toluène, le xylène, la tétraline, la décaline, des coupes pétrolières ou un mélange de ceux-ci.

25. Procédé de préparation selon l'une quelconque des revendications 21 à 24, **caractérisé en ce que** le polymère structurant c est tel que défini aux revendications 6, 7, 12 ou 13, le solvant dₛ est tel que défini aux revendications 8 et 14, les agents réticulants d_{r r} ou d_{r i} sont tels que définis aux revendications 9 et 15 et la substance chimique active x est telle que définie aux revendications 16 à 18.

26. Dispersion, apte à être utilisée pour la préparation d'un film d'élastomère selon l'une quelconque des revendications 1 à 20, **caractérisée en ce qu'**elle comprend :
- une phase A contenant un élastomère dissous dans un solvant organique a (phase hydrophobe ou apolaire), tel que défini à la revendication 24, dans laquelle est dispersée, sous forme d'un gel réversible ou non-réversible, une phase B comprenant une substance chimique active x incluse dans un polymère structurant c, un solvant dₛ, et éventuellement un agent réticulant d_{r r} ou d_{r i},.

27. Procédé de préparation d'une dispersion selon la revendication 26, **caractérisé en ce qu'**il comprend :
- la préparation d'une phase A, par dissolution de l'élastomère dans un solvant organique a ;
- la préparation d'une phase B (gel), selon l'une des modalités suivantes, selon le type de gel :
∗ dans le cas où le gel est réticulé de manière réversible, c'est à dire présente une température de transition Ttrans gel-liquide, la phase B s'obtient :
- soit par mélange d'au moins une substance chimique active x dans un solvant dₛ, suivi du mélange de la solution obtenue avec un polymère structurant c, à une température supérieure à la température de transition Ttrans, correspondant à la transition gel-liquide, laquelle température Ttrans est comprise entre 20°C et 120°C, puis dispersion de cette phase B dans la phase A à une température supérieure à Ttrans ;
- soit par mélange d'au moins une substance chimique active x dans un solvant dₛ, suivi du mélange de la solution obtenue avec un polymère structurant c, puis la dispersion de cette phase B dans la phase A, avant ajout de l'agent réticulant d_{r r}.
∗ dans le cas où le gel est réticulé de manière non-réversible, la phase B s'obtient par mélange d'au moins une substance chimique active x dans un solvant dₛ, suivi du mélange de la solution obtenue avec un polymère structurant c, puis la dispersion de cette phase B dans la phase A, avant ajout de l'agent réticulant dᵣ ᵢ.

28. Application du film d'élastomère selon l'une quelconque des revendications 1 à 20 en tant que revêtement de supports.

29. Gant, **caractérisé en ce qu'**il est revêtu d'un film d'élastomère selon l'une quelconque des revendications 1 à 20.

30. Doigtier, **caractérisé en ce qu'**il est revêtu d'un film d'élastomère selon l'une quelconque des revendications 1 à 20.

31. Préservatif, **caractérisé en ce qu'**il est revêtu d'un film d'élastomère selon l'une quelconque des revendications 1 à 20.

## Claims

1. Elastomer film containing at least one active chemical substance in the form of a phase which is dispersed in the said elastomer, which film is **characterized in that** the said disperse phase is in the form of a gel, reversible or irreversible as a function of the temperature, and filled with active chemical substance x.

2. Elastomer film according to Claim 1, **characterized in that**, when the said gel is reversible as a function of the temperature, it, has a gel-liquid transition temperature (Ttrans) of between 0°C and 120°C, preferably between 20°C and 70°C.

3. Elastomer film according to Claim 1 or Claim 2, **characterized in that** the said reversible gel consists essentially of a structuring polymer c, of a solvent dₛ and of the active chemical substance x.

4. Elastomer film according to Claim 3, **characterized in that** it additionally contains a reversible crosslinking agent d_{rc}.

5. Elastomer film according to Claim 3 or Claim 4, **characterized in that** the gel includes:
- between 25 and 97 % (mass content) of solvent dₛ, relative to the gel,
- between 1 and 74 % (mass content) of structuring polymer c, relative to the gel,
- between 1 and 74 % (mass content) of the said active chemical substance x, relative to the gel, and
- between 0 and 1 % of crosslinker d_{r c} (crosslinker mass content/total constituents).

6. Elastomer film according to any one of Claims 1 to 5, **characterized in that** the structuring polymer c is of hydrophilic type and immiscible with the elastomer phase.

7. Elastomer film according to any one of Claims 1 to 6, **characterized in that** the said structuring polymer c is selected from the group consisting either of the crystallizable or partially crystallizable polymers like, for example, polyethers, preferably from polyoxyethylenes, of molecular mass included between 1500 and 10⁶ daltons and polyoxyethylene-based polymers exhibiting, in the molten state, a miscibility with the solvent dₛ, or of polymers which are not organized in the solid state, like, for example, the polymers of natural origin like gelatin, gums, pectins and alginates, as well as some cellulose derivatives like methyl cellulose, hydroxyethyl cellulose, hydroxypropyl cellulose and carboxymethyl cellulose and synthetic compounds like polyethyleneimine, polyacrylic acid, polyacrylates and polymethacrylates such as poly(hydroxyethyl methacrylate), polyvinyl alcohol and its derivatives and polyvinyl ethers like poly(vinyl methyl ether).

8. Elastomer film according to any one of Claims 1 to 7, **characterized in that** the solvent dₛ is selected from the group consisting of polyols, preferably from ethylene glycol, glycerine and polyethylene glycols which are liquid at ambient temperature and of molecular mass between 62 (ethylene glycol) and 750 daltons (PEG750) and water, employed alone or as a mixture.

9. Elastomer film according to any one of Claims 1 to 8, **characterized in that** the crosslinker d_{rc} is selected from the group consisting of certain derivatives of boron, like boric acid or borax, of aluminium, like alumina hydrate or certain aluminium hydroxides and of certain divalent metals like zinc or calcium.

10. Elastomer film according to Claim 1, **characterized in that**, when the said gel is of irreversible type, it consists essentially of a structuring polymer c, of a solvent dₛ, of a crosslinker d_{ic} and of at least one active chemical substance x.

11. Elastomer film according to Claim 10, **characterized in that** the gel includes:
- between 25 and 97 % (mass content) of solvent dₛ, relative to the gel,
- between 1 and 74 % (mass content) of structuring polymer c, relative to the gel,
- between 1 and 74 % (mass content) of active chemical substance x relative to the gel, and
- between 0.0001 and 1 % of crosslinking agent d_{ic} (crosslinker/total constituents mass ratio).

12. Elastomer film according to Claim 10, **characterized in that** the structuring polymer c is of hydrophilic type and immiscible with the elastomer phase.

13. Elastomer film according to any one of Claims 10 to 12, **characterized in that** the said structuring polymer c is selected from the group consisting of the polymers of natural origin like gelatin, gums, pectins and alginates, or certain cellulose derivatives like methyl cellulose, hydroxyethyl cellulose, hydroxypropyl cellulose and carboxymethyl cellulose and of synthetic compounds like polyethyleneimine, polyacrylic acid, polyacrylates and polymethacrylates such as poly(hydroxyethyl methacrylate), polyvinyl alcohol and its derivatives and poly(vinyl ethers) like poly(vinyl methyl ether).

14. Elastomer film according to any one of Claims 10 to 13, **characterized in that** the compound dₛ is selected from the group consisting of polyols, preferably from ethylene glycol, glycerine and more generally polyethylene glycols which are liquid at ambient temperature and of molecular mass of between 62 (ethylene glycol) and 750 daltons (PEG750) and water, which are employed alone or as a mixture.

15. Elastomer film according to any one of Claims 10 to 14, **characterized in that** the crosslinker d_{i c} is selected from the group consisting of aldehydes or dialdehydes of low or high molecular weight and ureaformaldehyde and melamin-formaldehyde derivatives.

16. Elastomer film according to any one of Claims 1 to 15, **characterized in that** the said active chemical substance x is selected from compounds capable of causing a virtually instantaneous denaturing of proteins by simple contact, either by chemical reaction or by a physicochemical effect, such as a change in the surface tension.

17. Elastomer film according to Claim 16, **characterized in that** the said active chemical substance x is selected from the biocides.

18. Elastomer film according to Claim 16 or Claim 17,
**characterized in that** the said biocide is a quaternary ammonium, preferably dimethyldidecylammonium, biguanides, phthalaldehyde, phenolic derivatives, formaldehyde, nonionic surfactants containing at least one polyoxyethylene block, hexamidine, iodine compounds, nonionic surfactants with virucidal activity and sodium and potassium dichromates and hypochlorites, which are employed alone or as a mixture.

19. Elastomer film according to any one of Claims 1 to 18, **characterized in that** the elastomer is selected from polybutadiene, polyisoprene and SBR (*styrene* butadiene *rubber*), NBR (*nitrite butadiene rubber*), SBS (*styrene butadiene styrene*), SIS (*styrene isoprene styrene*) or SEBS (*styrene ethylene butylene styrene*) copolymers.

20. Elastomer film according to any one of Claims 1 to 19, **characterized in that** the mass proportions of the elastomer and of the gel are the following:
- elastomer: between 20 and 95 % (mass content), expressed relative to the final film, and
- gel: between 5 and 80 % (mass content), expressed relative to the final film.

21. Process for the preparation of the elastomer film according to any one of Claims 1 to 20, **characterized in that** it includes:
1) **the preparation of a dispersion of a gel, which includes:**
- the preparation of a phase A by dissolving the elastomer in an organic solvent a;
- the preparation of a phase B (gel) and the dispersion of the phase B in the phase A, according to one of the following methods:
when the gel is crosslinked reversibly, the phase B can be prepared according to two different ways, according to whether crosslinker d_{rc} is or is not employed:
(i) in the case where the gel is formed spontaneously by bonds of physical type, the phase B is prepared by mixing at least one active chemical substance x in a solvent dₛ, followed by mixing of the solution obtained with a polymer c, at a temperature higher than the transition temperature Ttrans, corresponding to the gel-liquid transition, which temperature Ttrans is between 20°C and 120°C, followed by the dispersion of the phase B in the phase A, at a temperature higher than Ttrans;
(ii) in the case where the addition of a crosslinker d_{rc} is necessary for the formation of the reversible gel, the phase B is prepared by mixing at least one active chemical substance x in a solvent dₛ, followed by mixing of the solution obtained with a structuring polymer c, then by the dispersion of this phase B in the phase A before addition of the crosslinker d_{rc};
when the gel is crosslinked irreversibly, the phase B is obtained by mixing at least one active chemical substance x in a solvent dₛ, followed by mixing of the solution obtained with a structuring polymer c, and then this phase B is dispersed in the phase A before addition of the crosslinking agent d_{ic} and
2) evaporation of the organic solvent a, to obtain an elastomer film enclosing gel inclusions in a stable dispersion form.

22. Process of preparation according to Claim 21, **characterized in that**, in the case of a gel crosslinked reversibly, the evaporation of the solvent is performed at a temperature lower than Ttrans.

23. Process of preparation according to Claim 21, **characterized in that**, in the case of a gel crosslinked irreversibly, the evaporation of the solvent is performed at a temperature chosen as a function of the desired evaporation time and included between 0 and 120°C.

24. Process according to any one of Claims 21 to 23, **characterized in that** the apolar or weakly polar solvent a is selected from aromatic, aliphatic and alicyclic hydrocarbons, for example paraffinic hydrocarbons, cyclohexane, benzene, toluene, xylene, tetralin, decalin, petroleum cuts or a mixture of these.

25. Process of preparation according to any one of Claims 21 to 24, **characterized in that** the structuring polymer c is as defined in Claims 6, 7, 12 or 13, the solvent dₛ is as defined in Claims 8 and 14, the crosslinking agents d_{rc} or d_{ic} are as defined in Claims 9 and 15 and the active chemical substance x is as defined in Claims 16 to 18.

26. Dispersion capable of being employed for the preparation of an elastomer film according to any one of Claims 1 to 20, **characterized in that** it includes:
- a phase A containing an elastomer dissolved in an organic solvent a (hydrophobic or apolar phase) as defined in Claim 24, in which is dispersed, in the form of a reversible or irreversible gel, a phase B including an active chemical substance x included in a structuring polymer c, a solvent dₛ, and optionally a crosslinking agent d_{rc} or d_{ic}.

27. Process for the preparation of a dispersion according to Claim 26, **characterized in that** it includes:
- the preparation of a phase A by dissolving the elastomer in an organic solvent a;
- the preparation of a phase B (gel), according to one of the following methods, according to the type of gel:
in the case where the gel is crosslinked reversibly, that is to say exhibits a gel-liquid transition temperature Ttrans, the phase B is obtained:
- either by mixing at least one active chemical substance x in a solvent dₛ, followed by mixing of the solution obtained with a structuring polymer c, at a temperature higher than the transition temperature Ttrans, corresponding to the gel-liquid transition, which temperature Ttrans is between 20°C and 120°C, then by dispersion of this phase B in the phase A at a temperature higher than Ttrans;
- or by mixing at least one active chemical substance x in a solvent dₛ, followed by mixing of the solution obtained with a structuring polymer c, then by dispersion of this phase B in the phase A before addition of the crosslinking agent d_{rc};
in the case where the gel is crosslinked irreversibly, the phase B is obtained by mixing at least one active chemical substance x in a solvent dₛ, followed by mixing the solution obtained with a structuring polymer c; this phase B is then dispersed in the phase A before addition of the crosslinking agent d_{ic}.

28. Application of the elastomer film according to any one of Claims 1 to 20 as coating for substrates.

29. Glove **characterized in that** it is coated with an elastomer film according to,any one of Claims 1 to 20.

30. Fingerstall **characterized in that** it is coated with an elastomer film according to any one of Claims 1 to 20.

31. Sheath or diaphragm **characterized in that** it is coated with an elastomer film according to any one of Claims 1 to 20.

## Patentansprüche

1. Elastomerfilm, welcher mindestens eine aktive chemische Substanz in Form einer in dem Elastomer dispergierten Phase enthält, wobei der Film **dadurch gekennzeichnet ist, daß** die dispergierte Phase in Form eines in Abhängigkeit von der Temperatur reversiblen bzw. nicht reversiblen Gels vorliegt und mit der aktiven chemischen Substanz x gefüllt ist.

2. Elastomerfilm nach Anspruch 1, **dadurch gekennzeichnet, daß** das Gel, wenn es in Abhängigkeit von der Temperatur reversibel ist, eine Gel-Flüssigkeit-Übergangstemperatur (Ttrans) besitzt, die zwischen 0°C und 120°C, vorzugsweise zwischen 20°C und 70°C liegt.

3. Elastomerfilm nach Anspruch 1 oder Anspruch 2, **dadurch gekennzeichnet, daß** das reversible Gel im wesentlichen aus einem strukturbildenden Polymer c, einem Lösungsmittel dₛ und der aktiven chemischen Substanz x besteht.

4. Elastomerfilm nach Anspruch 3, **dadurch gekennzeichnet, daß** er des weiteren ein Mittel d_{r r} zur reversiblen Vernetzung enthält.

5. Elastomerfilm nach Anspruch 3 oder Anspruch 4, **dadurch gekennzeichnet, daß** das Gel aufweist:
- zwischen 25 und 97% (Masseanteil) Lösungsmittel dₛ bezogen auf das Gel,
- zwischen 1 und 74% (Masseanteil) strukturbildendes Polymer c bezogen auf das Gel,
- zwischen 1 und 74% (Masseanteil) der aktiven chemischen Substanz x bezogen auf das Gel, und
- zwischen 0 und 1% Vernetzungsmittel d_{r r} (Masseanteil Vernetzungsmittel/Bestandteile insgesamt).

6. Elastomerfilm nach einem der Ansprüche 1 bis 5, **dadurch gekennzeichnet, daß** das strukturbildende Polymer c vom hydrophilen Typ und mit der Elastomerphase nicht mischbar ist.

7. Elastomerfilm nach einem der Ansprüche 1 bis 6, **dadurch gekennzeichnet, daß** das strukturbildende Polymer c aus der Gruppe ausgewählt ist, die entweder aus kristallisierbaren oder teilweise kristallisierbaren Polymeren wie beispielsweise Polyethern besteht, vorzugsweise unter den Polyoxyethylenen mit einer Molmasse von zwischen 1500 und 10⁶ Dalton und den Polymeren auf Basis von Polyoxyethylen, die im Schmelzezustand eine Mischbarkeit mit dem Lösungsmittel dₛ besitzen, oder aus im Festzustand nicht organisierten Polymeren besteht, wie beispielsweise Polymeren natürlichen Ursprungs wie Gelatine, Gummis, Pektinen und Alginaten sowie bestimmten Cellulosederivaten wie Methylcellulose, Hydroxyethylcellulose, Hydroxypropylcellulose und Carboxymethylcellulose und Syntheseverbindungen wie Polyethylenimin, Polyacrylsäure, Polyacrylaten und Polymethacrylaten wie etwa Polyhydroxyethylmethacrylat, Polyvinylalkohol und dessen Derivaten, Polyvinylethern wie etwa Polyvinylmethylether.

8. Elastomerfilm nach einem der Ansprüche 1 bis 7, **dadurch gekennzeichnet, daß** das Lösungsmittel dₛ aus der Gruppe ausgewählt ist, welche aus den Polyolen besteht, vorzugsweise unter Ethylenglycol, Glycerin und den Polyethylenglycolen, die bei Umgebungstemperatur flüssig sind und eine zwischen 62 (Ethylenglycol) und 750 Dalton (PEG750) liegende Molmasse besitzen, und Wasser, für sich oder in Mischung verwendet.

9. Elastomerfilm nach einem der Ansprüche 1 bis 8, **dadurch gekennzeichnet, daß** das Vernetzungsmittel d_{r r} aus der Gruppe ausgewählt ist bestehend aus bestimmten Borderivaten wie Borsäure oder Borax, Aluminiumderivaten wie hydriertem Aluminiumoxid oder bestimmten Aluminiumhydroxiden und bestimmten zweiwertigen Metatllen wie Zink oder Calcium.

10. Elastomerfilm nach Anspruch 1, **dadurch gekennzeichnet, daß** das Gel, wenn es vom nicht-reversiblen Typ ist, im wesentlichen aus einem strukturbildenden Polymer c, einem Lösungsmittel dₛ, einem Vernetzungsmittel d_{r i} und mindestens einer aktiven chemischen Substanz x besteht.

11. Elastomerfilm nach Anspruch 10, **dadurch gekennzeichnet, daß** das Gel aufweist:
- zwischen 25 und 97% (Masseanteil) Lösungsmittel dₛ bezogen auf das Gel,
- zwischen 1 und 74% (Masseanteil) strukturbildendes Polymer c bezogen auf das Gel,
- zwischen 1 und 74% (Masseanteil) aktive chemische Substanz x bezogen auf das Gel, und
- zwischen 0,0001 und 1% Vernetzungsmittel d_{r i} (Masseanteil Vernetzungsmittel/Bestandteile insgesamt).

12. Elastomerfilm nach Anspruch 10, **dadurch gekennzeichnet, daß** das strukturbildende Polymer c vom hydrophilen Typ und mit der Elastomerphase nicht mischbar ist.

13. Elastomerfilm nach einem der Ansprüche 10 bis 12, **dadurch gekennzeichnet, daß** das strukturbildende Polymer c aus der Gruppe ausgewählt ist, die aus Polymeren natürlichen Ursprungs wie Gelatine, Gummis, Pektinen und Alginaten oder aus bestimmten Cellulosederivaten wie Methylcellulose, Hydroxyethylcellulose, Hydroxypropylcellulose und Carboxymethylcellulose und Syntheseverbindungen wie Polyethylenimin, Polyacrylsäure, Polyacrylaten und Polymethacrylaten wie etwa Polyhydroxyethylmethacrylat, Polyvinylalkohol und dessen Derivaten, Polyvinylethern wie etwa Polyvinylmethylether besteht.

14. Elastomerfilm nach einem der Ansprüche 10 bis 13, **dadurch gekennzeichnet, daß** die Verbindung dₛ aus der Gruppe ausgewählt ist, welche aus den Polyolen besteht, vorzugsweise unter Ethylenglycol, Glycerin und allgemeiner den Polyethylenglycolen, die bei Umgebungstemperatur flüssig sind und eine zwischen 62 (Ethylenglycol) und 750 Dalton (PEG750) liegende Molmasse besitzen, und Wasser, für sich oder in Mischung verwendet.

15. Elastomerfilm nach einem der Ansprüche 10 bis 14, **dadurch gekennzeichnet, daß** das Vernetzungsmittel d_{r i} aus der Gruppe ausgewählt ist, welche aus den Aldehyden oder Dialdehyden mit geringem oder mit hohem Molekulargewicht, den Harnstoff-Formol- und Melamin-Formol-Derivaten besteht.

16. Elastomerfilm nach einem der Ansprüche 1 bis 15, **dadurch gekennzeichnet, daß** die aktive chemische Substanz x unter den Verbindungen ausgewählt ist, die in der Lage sind, eine quasi augenblickliche Denaturierung von Proteinen durch einfachen Kontakt zu bewirken, und zwar entweder durch chemische Umsetzung oder durch physiochemischen Effekt wie etwa eine Modifikation der Oberflächenspannung.

17. Elastomerfilm nach Anspruch 16, **dadurch gekennzeichnet, daß** die aktive chemische Substanz x unter den Biociden ausgewählt ist.

18. Elastomerfilm nach Anspruch 16 oder Anspruch 17, **dadurch gekennzeichnet, daß** das Biocid ein quaternäres Ammonium, vorzugsweise Dimethyldidecylammonium, Biguanide, Phthalaldehyd, phenolische Derivate, Formol, nicht-ionische Tenside mit mindestens einer Polyoxyethylensequenz, Hexamidin, iodhaltige Verbindungen, nicht-ionische Tenside mit Virucidwirkung, Natrium- und Kaliumbichromat und -hypochlorit ist, für sich oder in Mischung verwendet.

19. Elastomerfilm nach einem der Ansprüche 1 bis 18, **dadurch gekennzeichnet, daß** das Elastomer unter Polybutadien, Polyisopren, den Copolymeren SBR (*Styrene Butadiene Rubber),* NBR (*Nitrile Butadiene* Rubber), SBS (*Styrene Butadiene Styrene*), SIS (*Styrene Isoprene Styrene*) oder SEBS (*Styrene Ethylene Butylene Styrene*) ausgewählt ist.

20. Elastomerfilm nach einem der Ansprüche 1 bis 19, **dadurch gekennzeichnet, daß** die Masseanteile des Elastomers und des Gels die folgenden sind:
- Elastomer: zwischen 20 und 95% (Massegehalt), ausgedrückt in bezug auf den fertigen Film, und
- Gel: zwischen 5 und 80% (Massegehalt), ausgedrückt in bezug auf den fertigen Film.

21. Verfahren zur Herstellung des Elastomerfilms nach einem der Ansprüche 1 bis 20, **dadurch gekennzeichnet, daß** es umfaßt:
**1) die Herstellung einer Dispersion eines Gels, welche aufweist:**
- die Herstellung einer Phase A mittels Lösen des Elastomers in einem organischen Lösungsmittel a;
- die Herstellung einer Phase B (Gel) sowie das Dispergieren der Phase B in Phase A gemäß einer der folgenden Vorgehensweisen:
* falls das Gel reversibel vernetzt ist, kann die Phase B je nachdem, ob Vernetzungsmittel d_{r r} verwendet wird, auf zwei verschiedene Weisen hergestellt werden:
(i) falls sich das Gel spontan durch Bindungen vom physikalischen Typ bildet, wird die Phase B hergestellt durch Mischen mindestens einer aktiven chemischen Substanz x in ein Lösungsmittel dₛ, gefolgt vom Mischen der erhaltenen Lösung mit einem Polymer c bei einer Temperatur über der Übergangstemperatur Ttrans, welche dem Gel-Flüssigkeitsübergang entspricht, wobei die Temperatur Ttrans zwischen 20°C und 120°C liegt, gefolgt vom Dispergieren der Phase B in Phase A bei einer Temperatur über Ttrans;
(ii) falls die Zugabe eines Vernetzungsmittels d_{r r} für die Bildung des reversiblen Gels erforderlich ist, wird Phase B hergestellt durch Mischen mindestens einer aktiven chemischen Substanz x in ein Lösungsmittel dₛ, gefolgt vom Mischen der erhaltenen Lösung mit einem strukturbildenden Polymer c, daraufhin Dispergieren dieser Phase B in Phase A vor Zugabe des Vernetzungsmittels d_{r r};
* falls das Gel nicht-reversibel vernetzt ist, wird Phase B hergestellt durch Mischen mindestens einer aktiven chemischen Substanz x in ein Lösungsmittel dₛ, gefolgt vom Mischen der erhaltenen Lösung mit einem strukturbildenden Polymer c, woraufhin diese Phase B vor Zugabe des Vernetzungsmittels d_{r i} in Phase A dispergiert wird; und
2) **Abdampfen des organischen Lösungsmittels a zum** Herstellen eines Elastomerfilms, der Geleinschlüsse in Form einer stabilen Dispersion beinhaltet.

22. Herstellungsverfahren nach Anspruch 21, **dadurch gekennzeichnet, daß** im Fall eines reversibel vernetzten Gels das Abdampfen des Lösungsmittels bei einer niedrigeren Temperatur als Ttrans stattfindet.

23. Herstellungsverfahren nach Anspruch 21, **dadurch gekennzeichnet, daß** im Fall eines nicht-reversibel vernetzten Gels das Abdampfen des Lösungsmittels bei einer Temperatur stattfindet, die in Abhängigkeit von der gewünschten Verdampfungstemperatur gewählt wird und zwischen 0 und 120°C liegt.

24. Verfahren nach einem der Ansprüche 21 bis 23, **dadurch gekennzeichnet, daß** das apolare oder schwach polare Lösungsmittel a unter den aromatischen, aliphatischen und alicyclischen Kohlenwasserstoffen, z.B. paraffinischen Kohlenwasserstoffen, Cyclohexan, Benzol, Toluol, Xylol, Tetralin, Decalin, Erdölfraktionen oder einer Mischung von diesen ausgewählt ist.

25. Herstellungsverfahren nach einem der Ansprüche 21 bis 24, **dadurch gekennzeichnet, daß** das strukturbildende Polymer c der Definition in den Ansprüchen 6, 7, 12 oder 13 entspricht, das Lösungsmittel dₛ der Definition in den Ansprüchen 8 und 14 entspricht, die Vernetzungsmittel d_{r r} bzw. d_{r i} der Definition in den Ansprüchen 9 und 15 entsprechen, und die aktive chemische Substanz x der Definition in den Ansprüchen 16 bis 18 entspricht.

26. Dispersion, welche für die Verwendung zur Herstellung eines Elastomerfilms nach einem der Ansprüche 1 bis 20 geeignet ist, **dadurch gekennzeichnet, daß** sie aufweist:
- eine Phase A, welche ein Elastomer enthält, das in einem organischen Lösungsmittel a (hydrophobe oder apolare Phase) gemäß der Definition in Anspruch 24 gelöst ist, in welcher in Form eines reversiblen oder nicht-reversiblen Gels eine Phase B dispergiert ist, welche eine in einem strukturbildenden Polymer c eingeschlossene aktive chemische Substanz x, ein Lösungsmittel dₛ und gegebenenfalls ein Vernetzungsmittel d_{r r} bzw. d_{r i} aufweist.

27. Verfahren zur Herstellung einer Dispersion nach Anspruch 26, **dadurch gekennzeichnet, daß** es umfaßt:
- Herstellung einer Phase A durch Lösen des Elastomers in einem organischen Lösungsmittel a;
- Herstellung einer Phase B (Gel) nach einer der folgenden Vorgehensweisen je nach dem Typ des Gels:
* falls das Gel reversibel vernetzt ist, d.h. falls es eine Gel-Flüssigkeits-Übergangstemperatur Ttrans besitzt, wird Phase B hergestellt:
- entweder durch Mischen mindestens einer aktiven chemischen Substanz x in ein Lösungsmittel dₛ, gefolgt vom Mischen der erhaltenen Lösung mit einem strukturbildenden Polymer c bei einer Temperatur über der Übergangstemperatur Ttrans, welche dem Gel-Flüssigkeits-Übergang entspricht, wobei die Temperatur Ttrans zwischen 20°C und 120°C liegt, daraufhin Dispergieren dieser Phase B in Phase A bei einer Temperatur über Ttrans;
- oder durch Mischen mindestens einer aktiven chemischen Substanz x in ein Lösungsmittel dₛ, gefolgt vom Mischen der erhaltenen Lösung mit einem strukturbildenden Polymer c, daraufhin Dispergieren dieser Phase B in Phase A vor Zugabe des Vernetzungsmittels d_{r r};
* falls das Gel nicht-reversibel vernetzt ist, wird Phase B hergestellt durch Mischen mindestens einer aktiven chemischen Substanz x in ein Lösungsmittel dₛ, gefolgt vom Mischen der erhaltenen Lösung mit einem strukturbildenden Polymer c, daraufhin Dispergieren dieser Phase B in Phase A vor Zugabe des Vernetzungsmittels d_{r i}.

28. Anwendung des Elastomerfilms nach einem der Ansprüche 1 bis 20 als Überzug für Träger.

29. Handschuh, **dadurch gekennzeichnet, daß** er mit einem Elastomerfilm nach einem der Ansprüche 1 bis 20 überzogen ist.

30. Fingerling, **dadurch gekennzeichnet, daß** er mit einem Elastomerfilm nach einem der Ansprüche 1 bis 20 überzogen ist.

31. Präservativ, **dadurch gekennzeichnet, daß** es mit einem Elastomerfilm nach einem der Ansprüche 1 bis 20 überzogen ist.
